# EUROPÄISCHE PATENTSCHRIFT

(11) **EP 1 400 503 B1**
(45) Veröffentlichungstag und Bekanntmachung des Hinweises auf die Patenterteilung: **08.08.2007**
(21) Anmeldenummer: 03019908.7
(22) Anmeldetag: 02.09.2003
(51) Int. Cl.: C07C 29/17, C07C 31/135

(54) **Verfahren zur Herstellung von Trimethylcyclohexyl-alkan-3-olen mit einem hohen Anteil an trans-Isomeren**
Method for producing trimethylcyclohexyl-3-alkanols with a high portion of trans-isomers
Procédé de préparation de trimethylcyclohexyl-3-alcanols ayant une grande proportion de trans-isomères

(30) Priorität: 19.09.2002 DE 10243466
(43) Veröffentlichungstag der Anmeldung: 24.03.2004
(73) Patentinhaber: Symrise GmbH & Co. KG, 37603 Holzminden (DE)
(72) Erfinder: Schatkowski, Dietmar, 37627 Stadtoldendorf (DE); Pickenhagen, Wilhelm, 37671 Höxter (DE); Struwe, Hartmut, 37671 Stahle (DE); Panten, Johannes, 37671 Lüchtringen (DE); Schäfer, Klaus, 37639 Bevern (DE)
(74) Vertreter: Eisenführ, Speiser & Partner

(56) Entgegenhaltungen:
- DE-A- 2 455 761
- DE-A- 10 062 771
- DATABASE CA [Online] CHEMICAL ABSTRACTS SERVICE, COLUMBUS, OHIO, US; GVINTER, L. I. ET AL: "Changes in the selectivity of catalytic action during hydrogenation of the dienone group. IX. Hydrogenation of.beta.-ionone on nickel catalysts" retrieved from STN Database accession no. 83:43517 CA XP002258888 & IZVESTIYA AKADEMII NAUK SSSR, SERIYA KHIMICHESKAYA (1975), (3), 590-4 ,
- SAVOIA D ET AL: "ACTIVE METALS FROM POTASSIUM-GRAPHITE. AIR-OXIDIZED NICKEL-GRAPHITE AS A NEW SELECTIVE HYDROGENATION CATALYST" JOURNAL OF ORGANIC CHEMISTRY, AMERICAN CHEMICAL SOCIETY. EASTON, US, Bd. 46, 1981, Seiten 5344-5348, XP009016115 ISSN: 0022-3263

## Beschreibung

Die vorliegende Erfindung betrifft ein Verfahren zur Herstellung eines Trimethylcyclohexyl-alkan-3-ols der Formel A 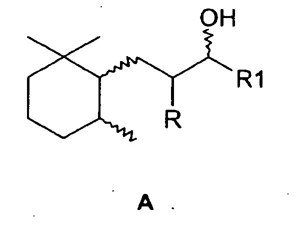 mit einem vorzugsweise hohen Anteil an trans-Isomeren der Formel D 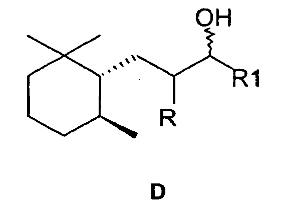 mit
R = H, Me, Et, n-Propyl, iso-Propyl, n-Butyl, iso-Butyl oder tert.-Butyl und
R1 = Me, Et, n-Propyl, iso-Propyl, n-Butyl, iso-Butyl oder tert.-Butyl, oder eines Gemisches mehrerer solcher Trimethylcyclohexyl-alkan-3-ole (die jeweils unter die Formel A fallen, wobei für jeden Mischungsbestandteil R und
R1 unabhängig voneinander und von den anderen Mischungsbestandteilen gewählt werden).

Verbindungen der Formel A sind wertvolle Riechstoffe, die wegen ihres charakteristischen, holzigen/ambrierten Geruchs sowie der sich aufgrund ihrer Struktur ergebenden guten fixierenden Eigenschaften und Wirkungen eine breite Verwendung in der Herstellung von Parfümkompositionen gefunden haben; vergleiche z.B. DE 24 55 761 A1 und DE 28 07 584 A1.

Die Trimethylcyclohexyl-alkan-3-ole der Formel A lassen sich u.a. durch Hydrierung der entsprechenden Verbindungen der Formel B 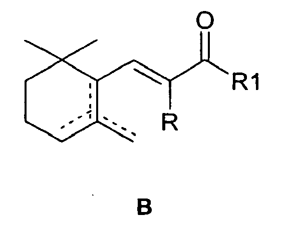 (in der R und R1 jeweils die entsprechende Bedeutung haben wie bereits zu Formel D bzw. A angegeben) erhalten.

Die in der Formel B eingezeichneten gestrichelten Linien stehen dabei für eine einzelne Doppelbindung, die in einer der drei eingezeichneten Positionen angeordnet sein kann. Unter Verwendung der für Jonone üblichen Nomenklatur handelt es sich also um eine *α-, β-* oder γ-ständige Doppelbindung (vergleiche Römpp-Lexikon Naturstoffe, Thieme, 1997, Seite 334 - 335).

Die dreifach ungesättigten Verbindungen der Formel B sind dabei auf dem Fachmann bekannte Weise z.B. durch (a) Kondensation von Citral mit einem geeigneten 2-Alkanon und (b) anschließende Cyclisierungsreaktion herstellbar; vergleiche z.B. Prelog et al. (Helv. Chim. Acta, 31, 417, 1948).

In der DE 24 55 761 C2 wird ausgeführt, dass bei der Hydrierung von Methyljononen (Verbindungen der Formel B mit R = Me) in Gegenwart von Raney-Nickel als alleinigem Katalysator nur 12 % der theoretisch möglichen Menge an Wasserstoff aufgenommen werden. Die Hydrierung führt also nicht zu Verbindungen der Formel A. Gemäß der DE 24 55 761 C2 führt eine Hydrierung jedoch dann zum gewünschten Produkt, wenn sie nicht in Gegenwart von Raney-Nickel allein, sondern in gleichzeitiger Gegenwart von Raney-Nickel und Kupferchromit durchgeführt wird.

Die DE 100 62 771 A1 schlägt vor, 1-(2,2,6-Trimethyl-1 bzw. 2-cyclohexen-1-yl)-1-alken-3-one (Verbindungen der Formel B) unter Verwendung von Ruthenium-Katalysatoren zu den entsprechenden 1-(2,2,6-Trimethylcyclohexyl)-3-alkanolen (Verbindungen der Formel A) mit einem hohen Gehalt an trans-Isomeren zu reduzieren. Die Hydrierung verläuft jedoch sehr langsam; im Beispiel 1 der DE 100 62 771 A1 ist eine Reaktionszeit von 60 Stunden angegeben, die für einen industriellen Prozess nicht akzeptabel wäre.

Es ist bereits aus der EP 0 118 809 B1 bekannt, dass die trans-Isomeren der Formel D sensorisch wertvoller sind als die entsprechenden cis-Verbindungen der Formel C 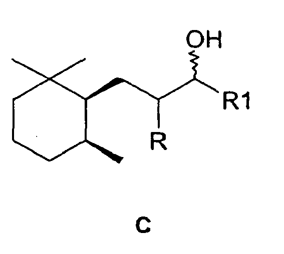 die bei der Reduktion von Verbindungen der Formel B immer im Gemisch mit den trans-Isomeren entstehen.

In der DE 28 07 584 A1 wird ohne Angabe der Isomeren-Verteilung auf 1-(2,6,6-Trimethylcyclohexyl)-hexan-3-ol (eine Verbindung der Formel A) verwiesen, welches nach totaler Hydrierung aus der entsprechenden Edukt-Verbindung der Formel B erhalten wird. Das Produkt wird von DRAGOCO unter dem Namen Timberol vertrieben, und in der späteren EP 0 118 809 B1 wird ausgeführt, dass Timberol nicht mehr als 10 bis 12 % der sensorisch wertvolleren trans-Verbindung enthält.

Neben der bereits diskutierten DE 100 62 771 A1 beschreiben auch eine Reihe anderer Veröffentlichungen die Herstellung von Verbindungen der Formel A mit einem möglichst hohen Anteil an trans-Isomeren der Formel D. Insbesondere sei insoweit auf die EP 0 118 817 A1 und die EP 0 456 932 B1 verwiesen. Die darin beschriebenen Verfahren sind jedoch entweder aufgrund der Verwendung schwierig zu handhabender Reagenzien wie z.B. Lithiumaluminiumhydrid oder aufgrund ihrer Vielstufigkeit nicht unmittelbar für eine Verwendung im technischen Maßstab geeignet sind.

Es war daher die Aufgabe der vorliegenden Erfindung, ein Verfahren zu entwickeln, dass eine Herstellung eines Trimethylcyclohexyl-alkan-3-ols mit einem vorzugsweise hohen Anteil an trans-Isomeren der Formel D oder eines Gemisches mehrerer solcher Verbindungen in nur einem Reaktionsschritt aus einem einfach und preiswert zugänglichen Edukt ermöglicht.

Vorzugsweise sollte das anzugebende Verfahren auch im großtechnischen Maßstab einfach und preiswert durchzuführen sein und nur eine geringe Reaktionszeit erfordern.

Weiter vorzugsweise sollten keine schwierig zu handhabenden Reagenzien eingesetzt werden.

Noch weiter vorzugsweise sollte das anzubietende Verfahren ohne größeren Aufwand so eingestellt werden können, dass der Anteil an trans-Isomeren der Formel D im Reaktionsprodukt zumindest 15 % beträgt, bezogen auf die Gesamtmenge an hergestellten trans- und cis-Isomeren.

Erfindungsgemäß wird diese Aufgabe gelöst durch ein Verfahren der eingangs genannten Art, in dem die entsprechende(n) Verbindung(en) der Formel B, in der R und R1 jeweils die eingangs angegebenen Bedeutungen haben, in Gegenwart eines Nickel-Katalysators, vorzugsweise von Raney-Nickel, und von Base, vorzugsweise Hydroxiden, Oxiden oder Carbonaten der Alkali- oder Erdalkalimetalle, katalytisch hydriert wird bzw. werden, wobei (im Unterschied insbesondere zur DE 24 55 761 C2) keine katalytisch aktiven Mengen an Kupferchromit vorhanden sind.

Unter den Verbindungen der Formel B, die in dem erfindungsgemäßen Verfahren eingesetzt werden können, seien exemplarisch die folgenden genannt:

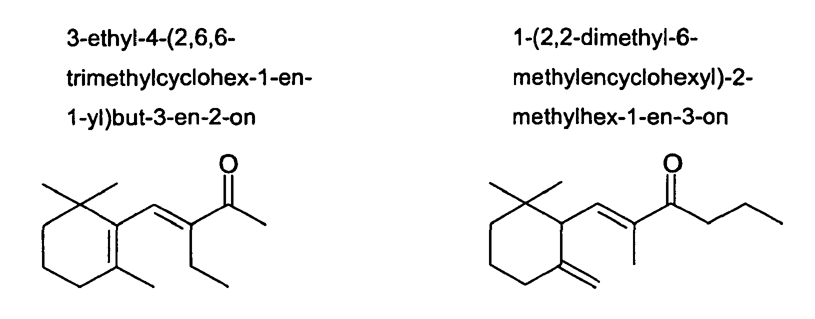

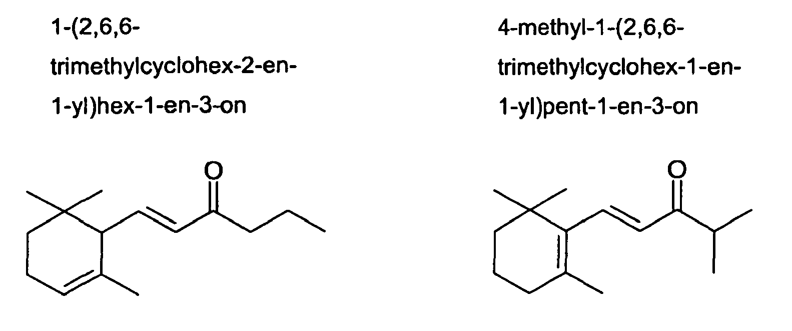

Die Gestaltung des erfindungsgemäßen Verfahrens ist dabei im Wesentlichen unabhängig davon, ob als Edukt ein Gemisch verschiedener Verbindungen der Formel B eingesetzt wird, so dass auch ein entsprechendes Gemisch mehrerer Trimethylcyclohexyl-alkan-3-ole entsteht, oder nur eine einzelne Verbindung der Formel B.

Überraschenderweise hat sich in umfangreichen eigenen Untersuchungen gezeigt, dass die von der Fachwelt angenommene Untauglichkeit der Verwendung von Raney-Nickel als alleinigem Katalysator, die insbesondere durch die entsprechenden Ausführungen in der DE 24 55 761 C2 dokumentiert sind, einer genaueren Betrachtung nicht standhält. Tatsächlich ist es vielmehr so, dass die vollständige Hydrierung von Verbindungen der Formel B in Gegenwart eines Nickel-Katalysators und von Base auch in Abwesenheit katalytisch aktiver Mengen an Kupferchromit möglich ist, dessen unbedingte Anwesenheit in der DE 24 55 761 C2 so vehement unterstrichen wird. Bei dem Nickel-Katalysator handelt es sich dabei vorzugsweise um Raney-Nickel, mit dem besonders gute Ausbeuten erreicht wurden.

Durch Variation einzelner Prozessparameter ließen sich überdies auch für den industriellen Maßstab taugliche Verfahrensbedingungen ermitteln, bei denen ein Trimethylcyclohexyl-alkan-3-ol der Formel A oder ein Gemisch mehrerer solcher Trimethylcyclohexyl-alkan-3-ole hergestellt wird, mit einem Anteil von zumindest 15 % trans-Isomeren der Formel D, bezogen auf die Gesamtmenge an hergestellten trans- und cis-Isomeren. Mit anderen Worten gilt für das Verhältnis der Stoffmengen von hergestellten trans- und cis-Isomeren nₜᵣₐₙₛ/n_{cis}≥ 15 : 85. Unter besonders bevorzugten Bedingungen gilt sogar: nₜᵣₐₙₛ/n_{cis} ≥ 35 : 65.

Besonders gute Resultate wurden erzielt bei Verwendung von Raney-Nickel in einer Menge von 0,001 bis 10 Gew.-%, vorzugsweise 0,1 bis 5 Gew.-%, bezogen auf die Masse der eingesetzten Verbindung(en) der Formel B, in der bzw. in denen R und R1 jeweils (für jeden Mischungsbestandteil unabhängig von den anderen) die angegebenen Bedeutungen haben. Wie erwähnt ist die Anwesenheit von Cu-Katalysatoren nicht erforderlich.

Besonders überraschend war, dass sich besonders positive Verfahrensergebnisse erzielen lassen, indem die Hydrierungsreaktion in Gegenwart von Base, vorzugsweise in Gegenwart von Hydroxiden, Oxiden oder Carbonaten der Alkali- oder Erdalkalimetalle durchgeführt wird. Insbesondere zeigte sich, dass die Anwesenheit von Basen die Entstehung der sensorisch wertvolleren trans-Isomeren zu fördern vermag. Die Konzentration der eingesetzten Base sollte dabei in Abhängigkeit von ihrer Basizität eingestellt werden. So lässt sich beispielsweise 1 g NaOH durch 0,8 g KOH oder 2,5 g Ca(OH)₂ ersetzen. Je nach Wahl der verwendeten Base hat sich der Einsatz basischer wässriger Lösungen bewährt, in denen das Massenverhältnis von Base zu Wasser im Bereich zwischen 0,1 : 100 und 50 : 100 lag.

Das Massenverhältnis von Base zu eingesetztem Katalysator (insbesondere Raney-Nickel) liegt dabei vorzugsweise im Bereich zwischen 0,01: 100 und 10 : 100, vorzugsweise im Bereich zwischen 1 : 100 und 3 : 100.

Vorzugsweise wird die katalytische Hydrierung bei einer Temperatur im Bereich zwischen 40 und 350 °C durchgeführt, wobei sich Temperaturen im Bereich zwischen 200 und 300 °C als besonders vorteilhaft erwiesen haben.

Der Wasserstoffdruck während der Hydrierung kann 1 - 200 bar betragen, bevorzugt ist ein Druckbereich von 10 - 50 bar.

Als besonders vorteilhaft hat sich herausgestellt, die Aufheizgeschwindigkeit des Reaktionsgemisches in Abhängigkeit von der Ansatzgröße zu justieren. Soll die katalytische Hydrierung im bevorzugten Temperaturbereich zwischen 200 und 300 °C durchgeführt werden, sollte die Aufheizgeschwindigkeit so gewählt werden, dass die gewünschte Reaktionstemperatur - ausgehend von einer Umgebungstemperatur von etwa 25 °C - innerhalb von etwa 5 bis 60 Minuten, vorzugsweise jedoch innerhalb 10 bis 15 Minuten erreicht ist. Durch eine derartige Maßnahme wird die Bildung der trans-Isomeren (Formel D) gegenüber der Bildung der cis-Isomeren (Formel C) begünstigt.

Insgesamt ist es vorteilhaft, die Hydrierung bei hohen Temperaturen und kurzen Reaktionszeiten durchzuführen. Eine Reaktionszeit im Bereich zwischen 0,5 und 3 Stunden ist bevorzugt. Die Verwendung eines Festbettreaktors ist in vielen Fällen vorteilhaft. Dies führt nicht nur zu einer deutlichen Erhöhung des Anteils an trans-Isomeren im Produktgemisch, sondern die hierdurch deutlich verbesserte Raum-/Zeit-Ausbeute führt auch insgesamt aufgrund der sehr kurzen Reaktionszeiten zu einer Schonung des Produktes, was sich nach erfolgter Reinigung in einer verbesserten sensorischen Qualität bemerkbar macht.

Ein weiterer Prozessparameter, dessen Optimierung insbesondere zu einer Verkürzung der Reaktionszeiten beitragen kann, ist die Rührgeschwindigkeit. So hat sich in Untersuchungen herausgestellt, dass sich in Versuchsautoklaven mit Begasungsrührer durch Veränderung der Rührgeschwindigkeit bei ansonsten gleichen Reaktionsbedingungen die gebildete Menge an trans-Isomeren der Formel D bei Erhöhung von 400 U/min. auf 1600 U/min. verdoppeln ließ. Gleichzeitig konnte die Reaktionszeit von 24 Stunden auf eine Stunde reduziert werden. Allgemein gilt, dass ein intensiver Stoffaustausch die Bildung der trans-Isomeren begünstigt. Bei Begasungsrührern führt beispielsweise eine Erhöhung der Drehzahl zu einem intensiveren Stoffaustausch, was mit einer erhöhten Löslichkeit des Wasserstoffs im Reaktionsmedium aufgrund einer besseren Durchmischung und einer besseren Verteilung des heterogenen Katalysators begründet wird. Anlagen, die wie z. B. sogenannte Loop-(Schleifen)-Reaktoren den intensiven Stoffaustausch zwischen fester, flüssiger und Gasphase begünstigen, führen ebenfalls zu günstigen Betriebsbedingungen und guten Raum/Zeit-Ausbeuten.

Es sei angemerkt, dass die Hydrierung sowohl in Substanz als auch in Lösung erfolgen kann. Als Lösungsmittel eignen sich hierbei insbesondere Alkohole wie Methanol, Ethanol, Ethylenglykol, Propylenglykol und deren Mischungen, Ester wie Ethylacetat und Kohlenwasserstoffe wie z.B. Hexan und Cyclohexan.

Es sei weiter angemerkt, dass die erfindungsgemäße Verwendung von NickelKatalysatoren wie Raney-Nickelin Gegenwart von Base nicht nur den Vorteil bietet, Reaktionsprodukte der Formel A mit einem hohen Anteil an trans-Isomeren der Formel D in einem einzigen Reaktionsschritt ausgehend von den leicht zugänglichen Verbindungen der Formel B zu liefern, sondern auch noch eine komplettere Umsetzung des Eduktes bewirkt. Der erhöhte Umsatz führt letztlich zu einer sensorisch verbesserten Qualität der Produkte und erleichtert deren Aufreinigung.

Gemäß, einem zweiten Aspekt betrifft die vorliegende Erfindung auch ein Verfahren zur Herstellung einer Parfümkomposition, mit folgenden Schritten:
- Herstellen eines Trimethylcyclohexyl-alkan-3-ols mit einem Anteil an trans-Isomeren der Formel D mit R = H, Me, Et, n-Propyl, iso-Propyl, n-Butyl, iso-Butyl oder tert.-Butyl und R1 = Me, Et, n-Propyl, iso-Propyl, n-Butyl, iso-Butyl oder tert.-Butyl, oder eines Gemisches mehrerer solcher Trimethylcyclohexyl-alkan-3-ole,
- gegebenenfalls Isolieren und/oder Reinigen des Trimethylcyclohexyl-alkan-3-ols oder des Gemisches,
- Vermischen einer sensorisch wirksamen Menge des Trimethylcyclohexylalkan-3-ols oder des Gemisches mit einem oder mehreren üblichen Parfümbestandteilen.

Es versteht sich hierbei, dass sämtliche das erfindungsgemäße Verfahren zum Herstellen des Trimethylcyclohexyl-alkan-3-ols oder des entsprechenden Gemisches betreffenden Ausführungen auch insoweit gelten.

Die Erfindung wird nachfolgend anhand von Beispielen näher erläutert.

### Beispiel 1

In einem Rührautoklaven mit Begasungsrührer werden 2500 g eines Gemisches aus Methyljonon, das die n-Methylionone 1-(2,6,6-Trimethylcyclohex-2-en-1-yl)pent-1-en-3-on (sogenanntes alpha-n-Methylionon) und 1-(2,6,6-Trimethylcyclohex-1-en-1-yl)pent-1-en-3-on (sogenanntes beta-n-Methylionon) in einem Verhältnis von ca. 4 : 1 enthält, bei 50 bar, mit 60 g, entsprechend 2,4 Gew.-% Raney-Nickel und unter Zusatz von 30 g NaOH 50%ig bei 1400 U/min. Rührgeschwindigkeit und einer Reaktionstemperatur von 260° - 280 °C innerhalb von 1,5 Std. hydriert. Die Aufheizung von Raumtemperatur auf die Reaktionstemperatur erfolgt in 25 Minuten. Nach Filtration und Destillation werden 2290 g vollständige hydriertes Produkt erhalten, das die 1-(2,2,6-Trimethylcyclohexyl)pentan-3-ole in einem trans/cis - Verhältnis von 1 : 3 enthält.

### Beispiel 2

In einem Rührautoklaven mit Begasungsrührer werden 2500 g eines Gemisches aus iso-Ethyljonon, das die iso-Ethyljonone 1-(2,6,6-Trimethylcyclohex-2-en-1-yl)5-methylhex-1-en-3-on und 1-(2,6,6-Trimethylcyclohex-1-en-1-yl)5-methylhex-1-3-on in einem Verhältnis von 3,5 : 1 enthält, mit 50 g Raney-Nickel, 25 g NaOH 20%ig in Wasser, bei 30 bar Wasserstoffdruck und einer Temperatur von 300 °C bei 1500 U/min. innerhalb von 45 Minuten vollständig hydriert. Die Aufheizung von Raumtemperatur auf die Reaktionstemperatur erfolgt in 15 Minuten. Nach Filtration und Destillation werden 2350 g vollständig hydriertes Produkt erhalten, das die 5-methyl-1-(2,2,6-Trimethylcyclohexyl)hexan-3-ole in einem trans/cis-Verhältnis von 1 : 2,1 enthält.

### Weitere Beispiele:

Die weiteren, in der nachfolgenden Tabelle 1 zusammengestellten Beispiele 3 bis 12 dienen der Illustration; die Parameter aus Beispiel 1 sind in der ersten Zeile der Tabelle angegeben. Die weiteren Beispiele wurden auf einem Versuchautoklaven mit Begasungsrührer durchgeführt. Der Übersichtlichkeit halber wurde jeweils ein Gemisch, wie in Beispiel 1 angegeben, eingesetzt.

Als Base wurde 50%ige Natronlauge eingesetzt. Versuche mit anderen, bereits weiter oben beschriebenen Basen, führten zu vergleichbaren Ergebnissen.

**Tabelle 1:**

| **Bsp.** | **Rühr. U/min** | **Druck H2** | **Reakt.-Temp.** | **Aufheizeitraum** | **RaNi [%]** | **Base [g]** | **Zeit¹ [min.]** | **trans/cis** |
|---|---|---|---|---|---|---|---|---|
| 1 | 1400 | 50 bar | 260°-280°C | 25 min. | 2,4 | 30 | 90 | 1 : 3,0 |
| 3 | 1200 | 30 bar | 180°C | 60 min. | 5,0 | 5 | 580 | 1 : 6,8 |
| 4 | 900 | 50 bar | 250°C | 30 min. | 3,0 | 3 | 150 | 1 : 4,8 |
| 5 | 1400 | 50 bar | 280°C | 15 min. | 2,5 | 5 | 60 | 1 : 2,9 |
| 6 | 1600 | 20 bar | 300°C | 15 min. | 2,0 | 2 | 45 | 1 : 2,0 |
| 7 | 1600 | 50 bar | 270°C | 12 min. | 2,0 | 4 | 50 | 1 : 2,1 |
| 8 | 1600 | 50 bar | 320°C | 17 min. | 1,5 | 3 | 40 | 1 : 1,8 |
| 9 | 1600 | 50 bar | 320°C | 17 min. | 1,5 | 1 | 40 | 1 : 2,0 |
| 10 | 900 | 30 bar | 280°C | 20 min. | 0,5 | 1 | 75 | 1 : 3,4 |
| 11 | 1400 | 10 bar | 330°C | 10 min. | 1,0 | 0,5 | 50 | 1 : 1,9 |
| 12 | 1400 | 10 bar | 330°C | 10 min. | 2,0 | 0,5 | 50 | 1 : 1,8 |

| | | | | | | | | |
|---|---|---|---|---|---|---|---|---|
| ¹Reaktionszeit bei Reaktionstemperatur | | | | | | | | |

## Patentansprüche

1. Verfahren zur Herstellung eines Trimethylcyclohexyl-alkan-3-ols mit einem Anteil an trans-Isomeren der Formel D 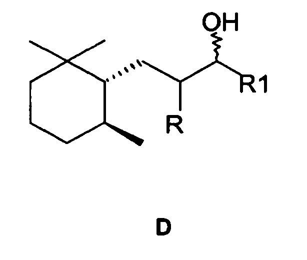 mit
R = H, Me, Et, n-Propyl, iso-Propyl, n-Butyl, iso-Butyl oder tert.-Butyl und
R1 = Me, Et, n-Propyl, iso-Propyl, n-Butyl, iso-Butyl oder tert.-Butyl, oder eines Gemisches mehrerer solcher Trimethylcyclohexyl-alkan-3-ole, wobei die entsprechende(n) Verbindung(en) der Formel B 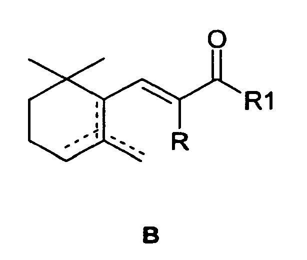 in der R und R1 jeweils die angegebenen Bedeutungen haben,
in Gegenwart eines Nickel-Katalysators, vorzugsweise von Raney Nickel, und von Base, vorzugsweise Hydroxiden, Oxiden öder Carbonaten der Alkali- oder Erdalkalimetalle, katalytisch hydriert wird bzw. werden, wobei keine katalytisch aktiven Mengen an Kupferchromit vorhanden sind.

2. Verfahren nach Anspruch 1, wobei die Verfahrensbedingungen so eingestellt sind, dass ein Trimethylcyclohexyl-alkan-3-ol oder ein Gemisch mehrerer solcher Trimethylcyclohexyl-alkan-3-ole hergestellt wird mit einem Anteil von zumindest 15 % trans-Isomer(en) der Formel D, bezogen auf die Gesamtmenge an hergestellten trans- und cis-Isomeren.

3. Verfahren nach einem der vorangehenden Ansprüche, **dadurch gekennzeichnet, dass** Raney Nickel in einer Menge von 0,001 bis 10 Gew.-%, vorzugsweise 0,1 bis 3 Gew.-%, eingesetzt wird, bezogen auf die Masse der eingesetzten Verbindung(en) der Formel B, in der bzw. in denen R und R1 jeweils die angegebenen Bedeutungen haben.

4. Verfahren nach einem der vorangehenden Ansprüche, **dadurch gekennzeichnet, dass** die katalytische Hydrierung bei einer Temperatur im Bereich zwischen 40 und 350 °C, vorzugsweise 200 und 300°C, durchgeführt wird.

5. Verfahren nach einem der vorangehenden Ansprüche, **dadurch gekennzeichnet, dass** die katalytische Hydrierung bei einem Druck im Bereich zwischen 1 und 200 bar, vorzugsweise 10-50 bar, durchgeführt wird.

6. Verfahren zur Herstellung einer Parfümkomposition, mit folgenden Schritten:
- Herstellen eines Trimethylcyclohexyl-alkan-3-ols mit einem Anteil an trans-Isomeren der Formel D 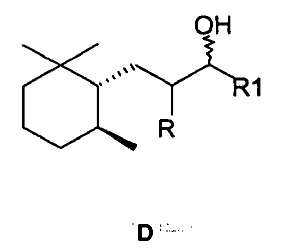 mit
R = H, Me, Et, n-Propyl, iso-Propyl, n-Butyl, iso-Butyl oder tert.-Butyl und
R1 = Me, Et, n-Propyl, iso-Propyl, n-Butyl, iso-Butyl oder tert.-Butyl, oder eines Gemisches mehrerer solcher Trimethylcyclohexyl-alkan-3-ole, gemäß einem der vorangehenden Ansprüche,
- gegebenenfalls Isolieren und/oder Reinigen des Trimethylcyclohexyl-alkan-3-ols oder des Gemisches,
- Vermischen einer sensorisch wirksamen Menge des Trimethylcyclohexyl-alkan-3-ols oder des Gemisches mit einem oder mehreren üblichen Parfümbestandteilen.

## Claims

1. A process for the production of a trimethylcyclohexylalkan-3-ol with a proportion of trans isomers of formula D 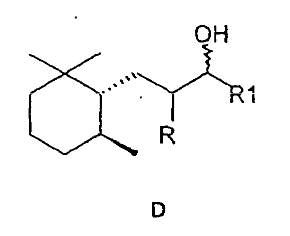 with
R = H, Me, Et, n-propyl, isopropyl, n-butyl, isobutyl or tert.-butyl and
R1 = Me, Et, n-propyl, isopropyl, n-butyl, isobutyl or tert.-butyl,
or of a mixture of several such trimethylcyclohexyl-alkan-3-ols,
wherein the corresponding compound(s) of formula B 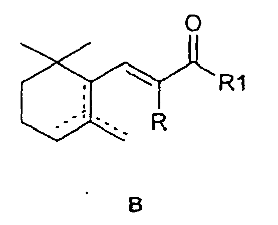 in which R and R1 each have the meanings stated, is/are catalytically hydrogenated in the presence of a nickel catalyst, preferably of Raney nickel, and of base, preferably hydroxides, oxides or carbonates of the alkali or alkaline earth metals, no catalytically active amounts of copper chromite being present.

2. The process according to claim 1, wherein the process conditions are adjusted such that a trimethylcyclohexylalkan-3-ol or a mixture of several such trimethylcyclohexylalkan-3-ols is produced with a proportion of at least 15% trans isomer(s) of formula D, based on the total amount of trans and cis isomers produced.

3. The process according to one of the above claims, **characterised in that** Raney nickel is used in a quantity of 0.001 to 10 wt.%, preferably 0.1 to 3 wt.%, based on the mass of the compound(s) of formula B used, in which R and R1 each have the meanings stated.

4. The process according to one of the above claims, **characterised in that** the catalytic hydrogenation is carried out at a temperature in the range of between 40 and 350°C, preferably 200 and 300°C.

5. The process according to one of the above claims, **characterised in that** the catalytic hydrogenation is carried out under a pressure in the range of between 1 and 200 bar, preferably 10 - 50 bar.

6. A process for the production of a perfume composition, having the following steps:
- production of a trimethylcyclohexylalkan-3-ol with a proportion of trans isomers of formula D 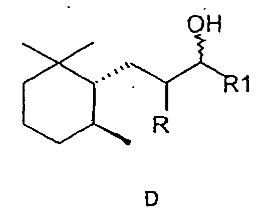 with
R = H, Me, Et, n-propyl, isopropyl, n-butyl, isobutyl or tert.-butyl and
R1 = Me, Et, n-propyl, isopropyl, n-butyl, isobutyl or tert.-butyl,
or of a mixture of several such trimethylcyclohexylalkan-3-ols, according to one of the above claims,
- optionally isolation and/or purification of the trimethylcyclohexylalkan-3-ol or of the mixture,
- mixing of a sensorially effective amount of the trimethylcyclohexylalkan-3-ol or of the mixture with one or more conventional perfume components.

## Revendications

1. Procédé de préparation d'un triméthylcyclohexylalcan-3-ol ayant une fraction d'isomère trans de formule D 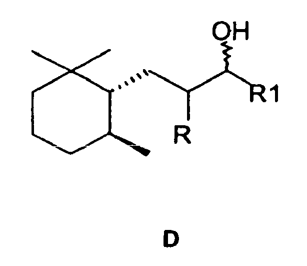 où
R = H, Me, Et, n-propyle, isopropyle, n-butyle, isobutyle ou tert-butyle et
R1 = Me, Et, n-propyle, isopropyle, n-butyle, isobutyle ou tert-butyle, ou d'un mélange de plusieurs de ces triméthylcyclohexylalcan-3-ols, dans lequel on soumet le ou les composés correspondants de formule B 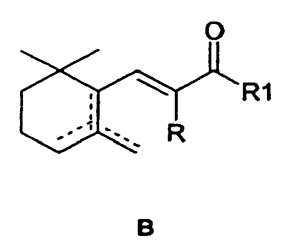 dans laquelle R et R1 ont chacun les significations indiquées,
à une hydrogénation catalytique en présence d'un catalyseur au nickel, de préférence du nickel de Raney, et d'une base, de préférence un hydroxyde, un oxyde ou un carbonate de métal alcalin ou de métal alcalino-terreux, aucune quantité catalytiquement active de chromite de cuivre n'étant présente.

2. Procédé selon la revendication 1, dans lequel on établit les conditions du procédé de manière à préparer un triméthylcyclohexylalcan-3-ol ou un mélange de plusieurs de ces triméthylcyclohexylalcan-3-ols ayant une fraction d'au moins 15 % d'isomère(s) trans de formule D par rapport à la quantité totale d'isomères cis et trans préparés.

3. Procédé selon l'une des revendications précédentes, **caractérisé en ce que** l'on utilise du nickel de Raney en une quantité de 0,001 à 10 % en masse, de préférence de 0,1 à 3 % en masse par rapport à la masse du ou des composés de formule B utilisés, dans lesquels R et R1 ont chacun les significations indiquées.

4. Procédé selon l'une des revendications précédentes, **caractérisé en ce que** l'hydrogénation catalytique s'effectue à une température se situant dans le domaine entre 40 et 350°C, de préférence entre 200 et 300°C.

5. Procédé selon l'une des revendications précédentes, **caractérisé en ce que** l'hydrogénation catalytique s'effectue sous une pression se situant dans le domaine entre 1 et 200 bar, de préférence de 10-50 bar.

6. Procédé de préparation d'une composition de parfum, comportant les étapes suivantes:
- préparation d'un triméthylcyclohexylalcan-3-ol ayant une fraction d'isomère trans de formule D 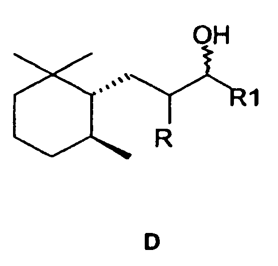
où
R = H, Me, Et, n-propyle, isopropyle, n-butyle, isobutyle ou tert-butyle et
R1 = Me, Et, n-propyle, isopropyle, n-butyle, isobutyle ou tert-butyle,
ou d'un mélange de plusieurs de ces triméthylcyclohexylalcan-3-ols, selon l'une des revendications précédentes,
- éventuellement isolement et/ou purification du triméthylcyclohexylalcan-3-ol ou du mélange,
- mélange d'une quantité sensoriellement efficace du triméthylcyclohexylalcan-3-ol ou du mélange avec un ou plusieurs constituants classiques de parfums.
